# EUROPEAN PATENT SPECIFICATION

(11) **EP 1 401 337 B1**
(45) Date of publication and mention of the grant of the patent: **15.06.2011**
(21) Application number: 02749617.3
(22) Date of filing: 20.06.2002
(51) Int. Cl.: A61B 17/11

(54) **ANASTOMOTIC DEVICE**
ANASTOMOSEVORRICHTUNG
DISPOSITIF ANASTOMOTIQUE

(30) Priority: 20.06.2001 US 299618 P
(43) Date of publication of application: 31.03.2004
(73) Proprietor: Park Medical, LLC, Nicholasville, KY 40356 (US)
(72) Inventor: PARK, Adrian, Edward, Nicholasville, KY 40356 (US); KNAPP, Charles, Francis, Georgetown, KY 40324 (US); CHARASH, William, Edward, Sudbury, MA 01776 (US); CUI, Hua, Lexington, KY 40514 (US)
(74) Representative: Bates, Philip Ian
(86) International application number: PCT/US2002/019566
(87) International publication number: WO 2003/000142

(56) References cited:
- WO-A-01/26582
- US-A- 5 376 376
- US-A- 5 540 712
- US-A- 5 720 776
- US-A- 6 007 544
- US-B1- 6 231 587

## Description

### Background of the Invention

### 1. Field of the Invention

The present invention is directed to gastrointestinal and enteric (including biliary) anastomoses and the like. The woven tube of wire of the invention is a three dimensional structure wherein the outer loops or ends of the woven tube fold or loop back in a manner which holds the luminal interface of the anastomotic site into apposition at the deployment site.

### 2. Description of the Related Art

Surgical procedures often require the joining (anastamosis) of two vessels or hollow vicera. For example, a permanent anastomosis between the stomach and intestine may be required in the performance of gastric bypass surgery for the morbidly obese as well as to alleviate blockage in the common bile duct by draining bile from the duct to the small intestine during surgery for pancreatic cancer. Surgical anastomosis generally involves manual suturing of the two structures. This process can be technically demanding and time consuming. This complex surgical procedure is even more challenging during minimally invasive surgery (MIS) where the surgeon is required to use instruments that are poorly designed for this task.

US 6,007,544 discloses a device for joining a wall of a blood vessel to an existing distal opening of a graft.

US-B-6231587 discloses an anastomosis device for side-to-side anastomosis with petals to compress and hold the walls of two adjacent lumens in opposition.

### Summary of the Invention

The present invention is directed to a woven tube of wire as defined in claim 1 for use in an automated anastomotic delivery device for surgery with special emphasis on MIS. The primary component is the woven tube of wire which deforms to make an anastomotic device when inserted into the walls of two adjacent vessels or lumens. The use of such a device for joining (anastomosing) two gastrointestinal or enteric (including biliary) vessels or lumens or the like is new.

The anastomotic delivery device is designed to allow the wire mesh tube to be slipped over a canula and pulled longitudinally, causing the tube to become longer and very small in diameter. After the wire mesh tube is loaded onto the canula, an outer sleeve is pushed over the tube up to the streamlined end of the delivery device, thereby providing a smooth surface for inserting into a vessel or lumen in the body. After the loaded canula is inserted into the appropriate vessel or lumen, a small sharp pointed wire, initially retracted in the center of the canula, is exposed at the tip (such as by pushing on a button in the handle) in order to assist the surgeon when passing the canula through the walls of the vessels or lumens. Once the canula/sleeve has penetrated both walls and is properly positioned, the outer sleeve is retracted. The wire mesh tube is constructed from a thermal, shape memory alloy such as nitinol such that when the sleeve is retracted, heat from the body causes the wire mesh tube to contract longitudinally to produce the anastomosis. This design eliminates the necessity for using a mechanical compression component in the delivery system and, therefore, reduces the complexity and size of the delivery system. Sufficient force is applied to the wall tissues such that the holes between the two lumens is enlarged (for drainage) and leakage outside the two lumens does not occur.

Additional objects, advantages and other novel features of the invention will be set forth in part in the description that follows and in part will become apparent to those skilled in the art upon examination of the foregoing or may be learned with the practice of the invention.

### Brief Description of the Drawings

Figure 1 is a front view of the woven tube of wire in its deployed form in which the outer loops or ends of the woven tube have deformed and everted to form petals which hold the walls of the lumens into apposition, the front and back petals of the deployed anastomotic device being shown as dark black and light gray lines, respectively.
Figure 2 is a side view of the woven tube prior to being slipped over a canula of the delivery device.
Figure 3A is a front view of the woven tube similar to Figure 1 with the walls of the lumens being omitted.
Figure 3B is a side view of the woven tube of Figure 3A.
Figure 4A shows the delivery device having the woven tube loaded and the sleeve pushed over the tube up to the end of the delivery device.
Figure 4B shows the delivery device inserted into a body cavity to a predetermined puncture site and further shows the tip of a wire, initially retracted in the canula, passed through the walls of the lumens.
Figure 4C shows the end of the delivery device passed through the walls of the lumens with the sheath partially retracted to expose the woven tube slipped over the canula.
Figure 4D shows the woven tube positioned at the juncture of the opposing puncture holes in the tissue, the walls of the lumens being held in a predetermined position guided by the delivery device.
Figure 4E shows the initial stage of deployment of the woven tube as the ends of the woven tube begin forming a petal configuration.
Figure 4F shows the woven tube in its deployed, flattened form gripping the walls of the lumens.
Figure 4G shows the delivery device being retracted from the body cavity through the opening in the flattened woven tube, the left-hand side portion of Figure 4G showing an end view of the deployed woven tube in the same manner as shown in Figure 1.

### Description of the Preferred Embodiments

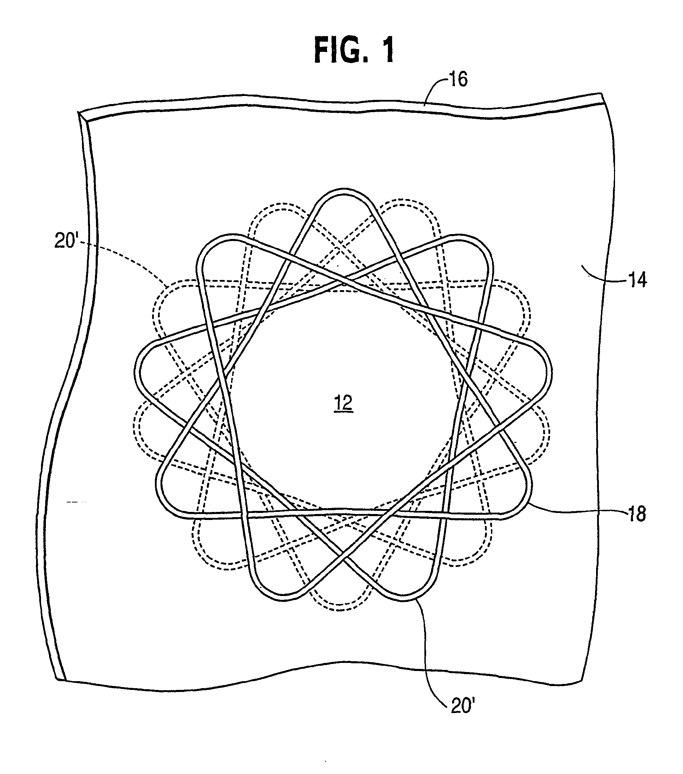

The tube 10 has an overlapping wire mesh design. The woven tube is designed to produce a round opening 12 between two layers of tissue 14, 16 and to hold the layers of tissue together for a watertight seal. The deployed anastomic device is essentially a woven tube 10 of wire 18 that is axially compressed as shown in Figure 1.

The woven tube 10 is defined by the wire diameter, number of circumferential and longitudinal openings or diamonds 20, the tube length and the center diameter. The openings or diamonds 20' at the longitudinal ends of the elongated woven tube are referred to as petals when the device is in the deployed shape (see Figure 1).

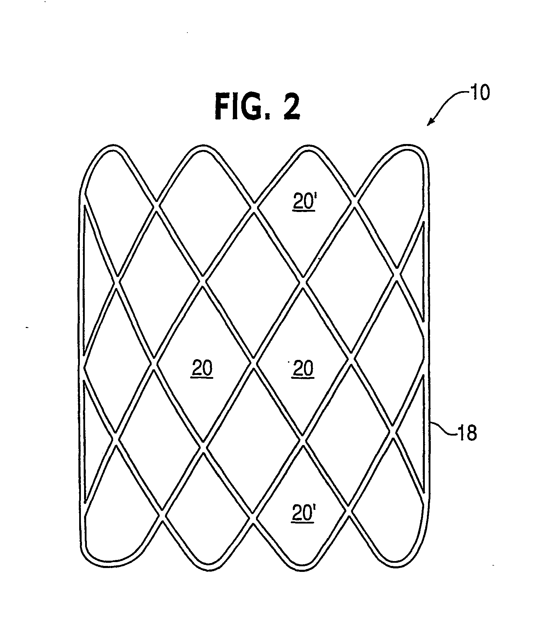

In use, the woven tube is forced into an elongated form (with much smaller diameter than that shown in Figure 2), placed through openings between the wall tissues of two lumens and allowed to return to the flattened shape of Figure 1. In the process, the tissues of both lumen walls are compressed between the petals of the flattened tube (see Figure 1) with the center diameter 12 of the flattened tube forming an opening between the lumens.

The woven tube can be applied, for example, through the common bile duct, and pushed through so that it connects the duct to the jejunum. After the connection has been made, the tube can be caused to deform and evert so that the ends spread out like the petals of a flower and form a connection between the two ducts. Since the tube is made of a wire mesh, scar tissue will grow around the flattened tube and eventually form a permanent connection.

The woven tube is made out of a shape memory metal. A shape memory metal is an alloy that changes its plasticity as heat is applied, allowing it to change shape. If a shape memory metal is annealed in a desired form (in a longitudinally compressed form), after it is reshaped (in a cylindrical tube form) it will return to its annealed shape (flattened form) if it is reheated at a significantly lower temperature. The very special property of thermal memory is especially helpful in the design of a low profile and flexible delivery system. The preferred shape memory metal is a titanium-nickel alloy, most preferably a nearly equiatomic alloy of titanium and nickel called nitinol. Specific nitinol alloys, which also have superelastic properties, can reshape at body temperature.

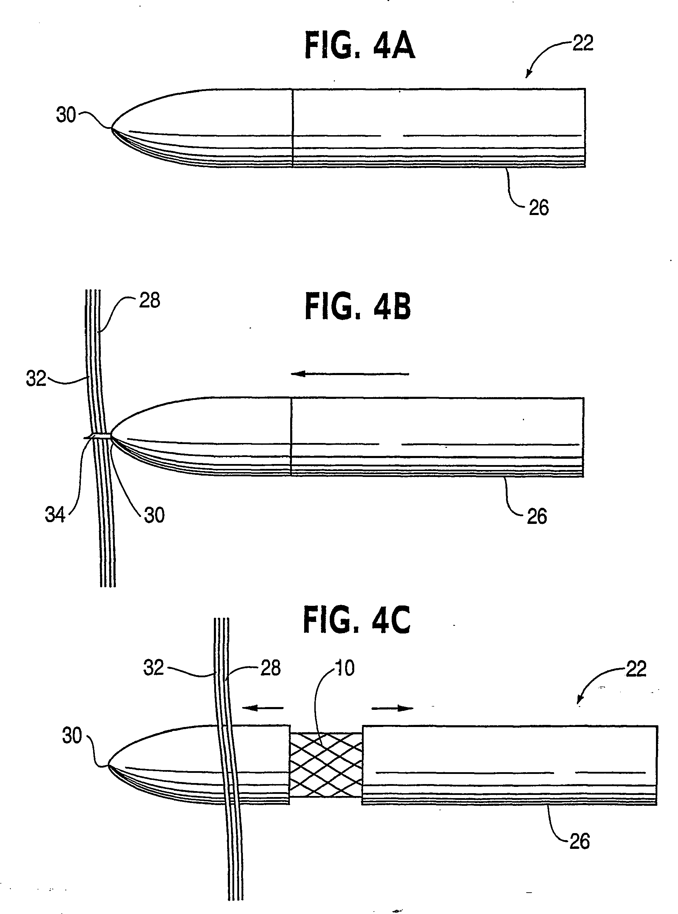

One embodiment of the delivery device 22 of the invention comprises a woven tube 10 mounted on a canula or delivery rod 24 covered by a retractable sheath 26 as shown in Figure 4A. In use in a side-to-side intestinal anastomosis, for example, the delivery device 22 is inserted into the body cavity through a trochar or tube (not shown) and the end 30 of the delivery device 22 is positioned at a predetermined puncture site in a first intestinal segment 28 either proximal or distal to the desired anastomotic site and the delivery device 22 is advanced intraluminally to the anastomotic site.

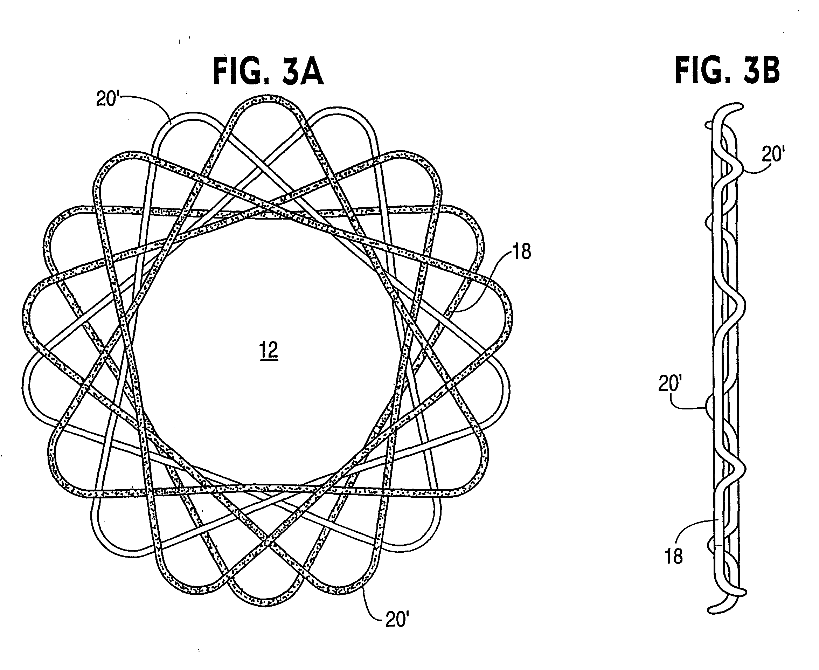

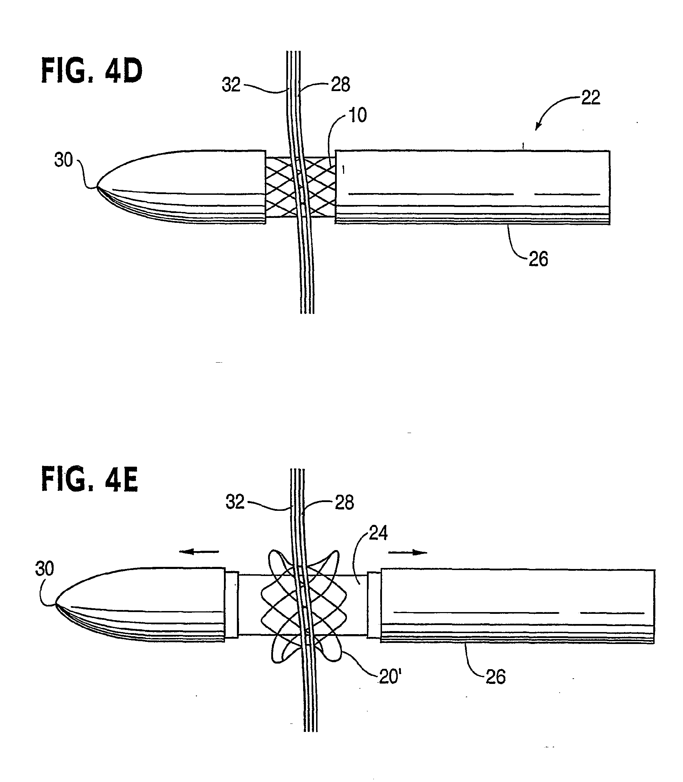

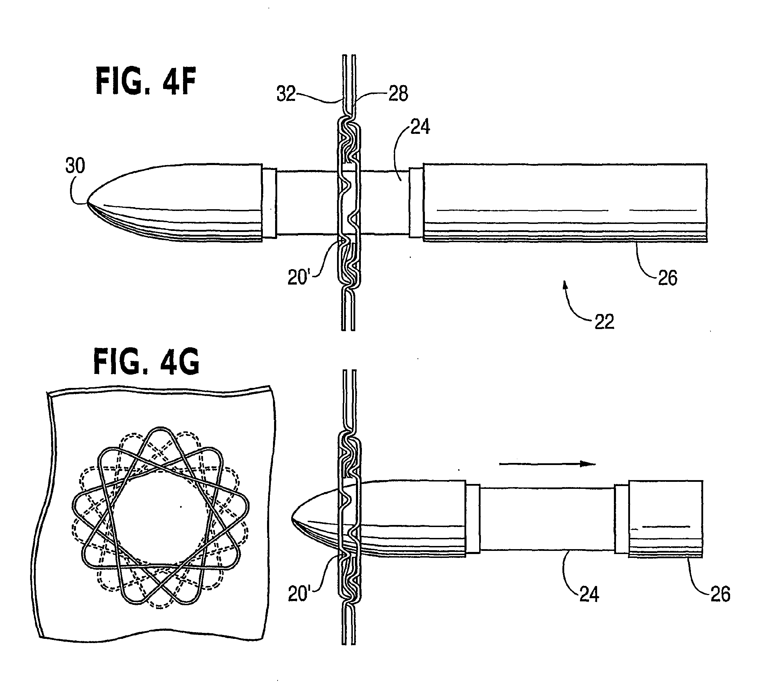

The second intestinal segment 32 is brought into close apposition to the first segment at the anastomotic site and the sharp tip of a wire 34, initially retracted in the center of the canula 24, is used to pierce through the wall of the first segment 28 and the wall of the second segment 32 and into the lumen of the second segment as shown in Figure 4B. The sheath 26 is retracted and the woven tube 10 is deployed as shown in the sequence of Figures 4C, 4D, 4E and 4F at the juncture of the apposing holes created by the tip of the wire 34 and assumes the petal configuration at the site to hold the two pieces of intestine in apposition. The woven tube deployed through two layers of intestine is shown in Figures 4F and 4G. The opposed petals 20 on opposite sides of the two layers of intestine 28, 32 are preferably interdigitated as shown in Figures 1, 3A and B and 4G.

The foregoing description of a preferred embodiment of the invention has been presented for purposes of illustration and description. It is not intended to be exhaustive or to limit the invention to the precise form disclosed. Obvious modifications or variations are possible in light of the above teachings. The embodiment was chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally and equitably entitled.

## Claims

1. An anastomotic device for joining of lumens or hollow viscera which is deployed at a luminal surface of two adjacent lumens, comprising a woven tube (10) of wire (18) with longitudinally spaced ends having an overlapping wire mesh design and having an unobstructed exterior and interior, the tube (10) being longitudinally extendable to cause the tube to become longer in length and smaller in diameter, the tube being constructed from a thermal, shape memory alloy having circumferentially spaced outer loops (20') at each of the longitudinally spaced ends of the woven tube of wire such that heat causes the tube as deployed to become larger in diameter and to contract longitudinally and become axially compressed and flattened and the outer loops (20') of the tube to deform and evert after being inserted into holes of walls of the two adjacent lumens, the loops of the tube as deployed thermally deforming and everting to form petals (20') which compresses and holds the walls of the two adjacent lumens in apposition while the exterior and interior of the woven tube of wire remains unobstructed, sufficient force being applied to the walls of the two adjacent lumens such that the holes between the two adjacent lumens are enlarged for drainage and leakage outside the two adjacent lumens is prevented solely by the deployed woven tube of wire.

2. The device of claim 1, wherein the thermal, shape memory alloy is a titanium nickel alloy.

3. An anastomotic delivery device having a streamlined end (30), comprising a woven tube (10) of wire (18) with longitudinally spaced ends having an overlapping wire mesh design and having an unobstructed exterior and interior, the tube (10) having circumferentially spaced outer loops (20') at each of the longitudinally spaced ends of the woven tube of wire, a canula (24) having an end designed to allow the tube (10) to be slipped over the canula (24) and pulled longitudinally causing the tube to become longer in length and smaller in diameter, an outer sleeve (26) adapted to be pushed over the tube up to the streamlined end of the device thereby providing a smooth surface for inserting through walls of two adjacent lumens at a luminal interface in a body, and subsequently retracted, a wire (34) having a tip, initially retracted in the canula, and adapted to be exposed at the tip to create holes in the walls of the two adjacent lumens to assist a surgeon when passing the device through the walls of the lumens, the tube (18) being constructed from a thermal, shape memory alloy such that when the sleeve is retracted heat from the body causes the tube to become larger in diameter and to contract longitudinally and become axially compressed and flattened and causes the outer loops (20') of the tube (18) to deform and evert to form petals (20') which compresses and holds the walls of the two adjacent lumens in apposition while the exterior and interior of the woven tube of wire remains unobstructed, sufficient force being applied to the walls of the two adjacent lumens such that the holes between the two adjacent lumens are enlarged for drainage and leakage outside the two adjacent lumens is prevented solely by the deployed woven tube of wire.

4. The device of claim 3, wherein the thermal, shape memory alloy is a titanium-nickel alloy.

5. The device of claim 1, wherein the petals are interdigitated.

6. The device of claim 3, wherein the petals are interdigitated.

7. The device of claim 5, wherein the thermal, shape memory alloy is a titanium-nickel alloy.

8. The device of claim 6, wherein the thermal, shape memory alloy is a titanium-nickel alloy.

## Patentansprüche

1. Anastomosenvorrichtung zum Verbinden von Lumen oder hohlen Viszera, die an einer luminalen Oberfläche von zwei nebeneinanderliegenden Lumen zum Einsatz kommt, umfassend eine gewebte Röhre (10) aus Draht (18) mit longitudinal beabstandeten Enden, die eine überlappende Drahtgeflechtkonstruktion und einen unbehinderten Außen- und Innenbereich aufweist, wobei die Röhre (10) der Länge nach erweiterbar ist, so dass die Röhre eine größere Länge und einen kleineren Durchmesser erhält, wobei die Röhre aus einer thermischen Formgedächtnislegierung konstruiert ist und umfangsmäßig voneinander beabstandete Außenschleifen (20') an jedem der longitudinal beabstandeten Enden der gewebten Drahtröhre hat, so dass Wärme bewirkt, dass die eingesetzte Röhre im Durchmesser größer wird und der Länge nach kontrahiert und axial komprimiert und abgeflacht wird und dass sich die Außenschleifen (20') der Röhre nach dem Einfügen in Löcher von Wänden der zwei nebeneinanderliegenden Lumen verformen und ausstülpen, wobei sich die Schleifen der eingesetzten Röhre thermisch verformen und ausstülpen, um Petalen (20') zu bilden, wodurch die Wände der zwei nebeneinanderliegenden Lumen in Apposition komprimiert und gehalten werden, während der Außen- und Innenbereich der gewebten Drahtröhre unbehindert bleiben, wobei ausreichend Kraft auf die Wände der zwei nebeneinanderliegenden Lumen aufgebracht wird, so dass die Löcher zwischen den zwei nebeneinanderliegenden Lumen für eine Drainage vergrößert werden und ein Auslaufen außerhalb der zwei nebeneinanderliegenden Lumen lediglich durch die eingesetzte gewebte Drahtröhre verhindert wird.

2. Vorrichtung nach Anspruch 1, wobei die thermische Formgedächtnislegierung eine Titan-Nickel-Legierung ist.

3. Anastomosenvorrichtung mit einem stromlinienförmigen Ende (30), die Folgendes umfasst: eine gewebte Röhre (10) aus Draht (18) mit longitudinal beabstandeten Enden, die eine überlappende Drahtgeflechtkonstruktion und einen unbehinderten Außen- und Innenbereich aufweist, wobei die Röhre (10) umfangsmäßig beabstandete Außenschleifen (20') an jedem der longitudinal beabstandeten Enden der gewebten Drahtröhre hat, eine Kanüle (24) mit einem Ende, das so gestaltet ist, dass die Röhre (10) über die Kanüle (24) geschoben und der Länge nach gezogen werden kann, so dass die Röhre eine größere Länge und einen kleineren Durchmesser erhält, eine Außenhülse (26), die so gestaltet ist, dass sie über die Röhre bis zum stromlinienförmigen Ende der Vorrichtung gedrückt werden kann, so dass eine glatte Oberfläche zum Einfügen durch Wände von zwei nebeneinanderliegenden Lumen an einer luminalen Grenzfläche in einem Körper entsteht, und anschließend zurückgezogen werden kann, einen Draht (34) mit einer Spitze, der zunächst in der Kanüle eingezogen und so gestaltet ist, dass er an der Spitze entblößt wird, um Löcher in den Wänden der zwei nebeneinanderliegenden Lumen zu erzeugen, um einen Chirurgen beim Führen der Vorrichtung durch die Wände der Lumen zu unterstützen, wobei die Röhre (18) aus einer thermischen Formgedächtnislegierung konstruiert ist, so dass, wenn die Hülse zurückgezogen ist, Wärme vom Körper bewirkt, dass die Röhre im Durchmesser größer wird und der Länge nach kontrahiert und axial komprimiert und abgeflacht wird, und bewirkt, dass sich die Außenschleifen (20') der Röhre (18) verformen und ausstülpen, um Petalen (20') zu bilden, wodurch die Wände der zwei nebeneinanderliegenden Lumen in Apposition komprimiert und gehalten werden, während der Außen- und Innenbereich der gewebten Drahtröhre unbehindert bleiben, wobei ausreichend Kraft auf die Wände der zwei nebeneinanderliegenden Lumen aufgebracht wird, so dass die Löcher zwischen den zwei nebeneinanderliegenden Lumen für eine Drainage vergrößert werden und ein Auslaufen außerhalb der zwei nebeneinanderliegenden Lumen lediglich durch die eingesetzte gewebte Drahtröhre verhindert wird.

4. Vorrichtung nach Anspruch 3, wobei die thermische Formgedächtnislegierung eine Titan-Nickel-Legierung ist.

5. Vorrichtung nach Anspruch 1, wobei die Petalen ineinandergreifen.

6. Vorrichtung nach Anspruch 3, wobei die Petalen ineinandergreifen.

7. Vorrichtung nach Anspruch 5, wobei die thermische Formgedächtnislegierung eine Titan-Nickel-Legierung ist.

8. Vorrichtung nach Anspruch 6, wobei die thermische Formgedächtnislegierung eine Titan-Nickel-Legierung ist.

## Revendications

1. Dispositif anastomotique pour joindre des lumières ou des viscères creux, lequel est déployé au niveau de la surface des lumières de deux lumières adjacentes, comprenant un tube tissé (10) en fil métallique (18) avec des extrémités espacées dans le plan longitudinal ayant une structure chevauchante en treillis de fil métallique et ayant un extérieur et un intérieur non obstrués, le tube (10) étant étirable dans le sens longitudinal pour faire que le tube devienne plus long en longueur et plus petit en diamètre, le tube étant fabriqué en un alliage à mémoire de forme thermique ayant des boucles extérieures espacées sur la circonférence (20') au niveau de chacune des extrémités espacées dans le plan longitudinal du tube tissé en fil métallique de sorte que la chaleur fait que le tube tel que déployé devienne plus grand en diamètre et se contracte dans le sens longitudinal et devienne axialement comprimé et aplati et que les boucles extérieures (20') du tube se déforment et font une éversion après avoir été insérées dans des trous des parois de deux lumières adjacentes, les boucles du tube tel que déployé se déformant thermiquement et faisant une éversion pour former des pétales (20') ce qui comprime et tient les parois des deux lumières adjacentes en apposition tandis que l'extérieur et l'intérieur du tube tissé en fil métallique demeurent non obstrués, une force suffisante étant appliquée aux parois des deux lumières adjacentes de sorte que les trous entre les deux lumières adjacentes sont élargis pour le drainage et une fuite hors des deux lumières adjacentes est empêchée uniquement par le tube tissé déployé en fil métallique.

2. Dispositif selon la revendication 1, dans lequel l'alliage à mémoire de forme thermique est un alliage de titane-nickel.

3. Dispositif de mise en place anastomotique ayant une extrémité fuselée (30), comprenant un tube tissé (10) en fil métallique (18) avec des extrémités espacées dans le plan longitudinal ayant une structure chevauchante en treillis de fil métallique et ayant un extérieur et un intérieur non obstrués, le tube (10) ayant des boucles extérieures espacées sur la circonférence (20') au niveau des extrémités espacées dans le plan longitudinal du tube tissé en fil métallique, une canule (24) ayant une extrémité conçue pour permettre de glisser le tube (10) sur la canule (24) et de le tirer dans le sens longitudinal faisant que le tube devienne plus long en longueur et plus petit en diamètre, un fourreau extérieur (26) adapté pour être poussé sur le tube jusqu'à l'extrémité fuselée du dispositif offrant ainsi une surface lisse pour l'insertion à travers les parois de deux lumières adjacentes au niveau d'une interface de lumières dans un corps, et être subséquemment rétracté, un fil métallique (34) ayant une pointe, initialement rétracté dans la canule et adapté pour être exposé au niveau de la pointe pour créer des trous dans les parois des deux lumières adjacentes afin d'aider un chirurgien lorsqu'il fait passer le dispositif à travers les parois des lumières, le tube (18) étant fabriqué en un alliage à mémoire de forme thermique de sorte que lorsque le fourreau est rétracté, la chaleur du corps fait que le tube devienne plus grand en diamètre et se contracte dans le sens longitudinal et devienne axialement comprimé et aplati et fait que les boucles extérieures (20') du tube (18) se déforment et font une éversion pour former des pétales (20'), ce qui comprime et tient les parois des deux lumières adjacentes en apposition tandis que l'extérieur et l'intérieur du tube tissé en fil métallique demeurent non obstrués, une force suffisante étant appliquée aux parois des deux lumières adjacentes de sorte que les trous entre les deux lumières adjacentes sont élargis pour le drainage et une fuite hors des deux lumières adjacentes est empêchée uniquement par le tube tissé déployé en fil métallique.

4. Dispositif selon la revendication 3, dans lequel l'alliage à mémoire de forme thermique est un alliage de titane-nickel.

5. Dispositif selon la revendication 1, dans lequel les pétales sont entrecroisés.

6. Dispositif selon la revendication 3, dans lequel les pétales sont entrecroisés.

7. Dispositif selon la revendication 5, dans lequel l'alliage à mémoire de forme thermique est un alliage de titane-nickel.

8. Dispositif selon la revendication 6, dans lequel l'alliage à mémoire de forme thermique est un alliage de titane-nickel.
